# EUROPEAN PATENT APPLICATION

(11) **EP 3 885 780 A1**
(43) Date of publication of application: **29.09.2021**
(21) Application number: 20165834.1
(22) Date of filing: 26.03.2020
(51) Int. Cl.: G01R 33/48, A61B 5/00, G01R 33/483, G01R 33/56, G01R 33/561

(54) **MAGNETIC RESONANCE IMAGING OF BREAST MICRO-CALCIFICATIONS**

(71) Applicant: Koninklijke Philips N.V., 5656 AG Eindhoven (NL)
(72) Inventor: NEELAVALLI, Jaladhar, 5656 AE Eindhoven (NL)
(74) Representative: Philips Intellectual Property & Standards

(57) **Abstract**

Disclosed herein is a method of medical imaging. The method comprises receiving (202) gradient echo magnetic resonance imaging data (123) descriptive of a breast region (319) of a subject (318). The method further comprises receiving (202) DIXON magnetic resonance imaging data descriptive of the breast region. The DIXON magnetic resonance imaging data and the gradient echo magnetic resonance imaging data are spatially matched. The method further comprises generating (204) a breast micro-calcification magnetic resonance image (128) by inputting the gradient echo magnetic resonance imaging data and the DIXON magnetic resonance imaging data into a breast micro-calcification image reconstruction module (122). The breast micro-calcification image reconstruction module is configured to output a breast micro-calcification magnetic resonance image in response to inputting the gradient echo magnetic resonance imaging data and the DIXON magnetic resonance imaging data. The breast micro-calcification magnetic resonance image is descriptive of a location of breast micro-calcifications, wherein the gradient echo magnetic resonance imaging data is a phase image.

## Description

### FIELD OF THE INVENTION

The invention relates to Magnetic Resonance Imaging, in particular to the magnetic resonance imaging of breast tissue.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the B0 field or the main magnetic field. Various quantities or properties of the subject can be measured spatially using MRI.

United States patent publication US 1,0401,459 B2 discloses methods for acquiring magnetic resonance images that accurately depict vascular calcifications, or other objects composed of magnetic susceptibility-shifted substances, in a subject are provided. The images are generally acquired using a pulse sequence that is designed to reduce physiological motion-induced artifacts and to mitigate chemical-shift artifacts from water-fat boundaries. Advantageously, the MRI technique described here suppresses chemical-shift artifacts without significantly reducing the signal intensity from fatty tissues, and thereby allows for more reliable visualization of vascular calcifications.

### SUMMARY OF THE INVENTION

The invention provides for a medical system, a computer program, and a method in the independent claims. Embodiments are given in the dependent claims.

Breast micro-calcifications are typically detected using a mammogram. This however exposes the subject to X-rays. Embodiments may provide a means of imaging breast micro-calcifications without the use of ionizing radiation. To achieve this, two types of magnetic resonance image data are used. The first is a gradient echo magnetic resonance imaging data and the second is DIXON magnetic resonance image data. In DIXON magnetic resonance imaging, measurements are taken at multiple phases and the image components can be separated into fat image which images the fat or lipid content of voxels and a water image which images non-adipose tissue. The DIXON magnetic resonance imaging data and the gradient echo magnetic resonance imaging data are input into a breast micro-calcification image reconstruction module which outputs a breast micro-calcification magnetic resonance image, which shows the breast micro-calcifications. The breast micro-calcification magnetic resonance image can be further filtered using a high-pass spatial filter and used to generate a projection image. The resulting projection image is comparable to a conventional mammogram for detecting breast micro-calcifications.

In one aspect the invention provides for a medical system that comprises a memory that stores machine-executable instructions and a breast micro-calcification image reconstruction module. The breast micro-calcification image reconstruction module is configured to output a breast micro-calcification magnetic resonance image in response to inputting a gradient echo magnetic resonance imaging data and DIXON magnetic resonance imaging data. The DIXON magnetic resonance imaging data may comprise both fat and water images. The breast micro-calcification phase magnetic resonance image is descriptive of a location of breast micro-calcifications. The gradient echo magnetic resonance imaging data is a phase image. The medical system further comprises a computational system that is configured for controlling the medical system.

Execution of the machine-executable instructions causes the computational system to receive a gradient echo magnetic resonance imaging data descriptive of a breast region of a subject. The breast region of the subject may for example be within a region of interest. Execution of the machine-executable instructions further causes the processor to receive DIXON magnetic resonance imaging data descriptive of the breast region. The DIXON magnetic resonance imaging data and the gradient echo magnetic resonance imaging data are spatially matched. The spatial matching means that there may either be a one-to-one correspondence between voxels of the gradient echo magnetic resonance imaging data and the Dixon magnetic resonance image or there may be a registration which provides a mapping between the voxels of the two.

In some examples a registration may be used to transform one of the gradient echo magnetic resonance imaging data and the DIXON magnetic resonance imaging data so that they match or are spatially matched with each other. However, in most cases the gradient echo magnetic resonance imaging data and the DIXON magnetic resonance imaging data will be acquired such that they are spatially matched by the acquisition. In some cases the gradient echo magnetic resonance imaging data and the acquisition of the DIXON magnetic resonance imaging data may be combined so that they are automatically spatially matched.

Execution of the machine-executable instructions further causes the computational system to generate the breast micro-calcification magnetic resonance image by inputting the gradient echo magnetic resonance imaging data and the DIXON magnetic resonance imaging data into the breast micro-calcification image reconstruction module. This embodiment may be beneficial because it may provide for a means of identifying breast micro-calcifications without the use of an X-ray machine.

The gradient echo magnetic resonance imaging data may have been acquired in several different ways. In some cases, it may be a single-echo long TE gradient echo magnetic resonance imaging data. In other cases, it may have been acquired using a multi-echo gradient echo magnetic resonance imaging protocol. In this case it may span several echo times. In the case of a multi-echo gradient the DIXON magnetic resonance imaging data and the gradient echo magnetic resonance imaging data may be acquired at the same time.

In another embodiment the gradient echo magnetic resonance imaging data is a so-called long TE or echo time gradient echo magnetic resonance imaging data. The TE or echo time value may be used to control how small the calcifications can be detected. The term long TE is also relative to the DIXON magnetic resonance imaging acquisition which uses a relatively short TE or echo time. One criterion for defining a long TE would be to make it comparable to or less than the T2* of fat tissue for the particular magnetic resonance imaging protocol. The T2* is dependent on the magnetic field strength and will therefore be dependent upon the particular magnetic resonance imaging system.

The phase of the gradient echo magnetic resonance imaging data may essentially be freely chosen. There are however some practical choices which may make the operation or the reconstruction of images easier. For example, the gradient echo magnetic resonance imaging data can be chosen to be in phase or out of phase with the fat signal. Being in phase with fat may be beneficial because the image is more uniform and numerically the image processing is easier. However, a knowledge of the DIXON magnetic resonance imaging data enables mapping to be performed effectively. In another embodiment, the breast micro-calcification magnetic resonance image is a phase image.

In another embodiment, the DIXON magnetic resonance imaging data is a water image.

In another embodiment execution of the machine-executable instructions further cases the computational system to calculate a filtered breast micro-calcification magnetic resonance image by applying a high-pass spatial filter to the breast micro-calcification magnetic resonance image. This embodiment may be beneficial because the larger structures of the breast micro-calcification magnetic resonance image may be removed. One means of implementing a high-pass spatial filter is to calculate an image that has been first low-pass filtered, which essentially blends out features with a high spatial frequency and then remove this from the original image.

In another embodiment execution of the machine-executable instructions further causes the computational system to generate a breast micro-calcification projection image by calculating a projection image of the filtered breast micro-calcification image. This embodiment may be beneficial because the breast micro-calcification image may for example be a three-dimensional dataset or be a stack of two-dimensional slices. The production of the projection image may make it easier for a physician who is accustomed to examining X-rays to interpret the data. The projection image may for example be calculated in different ways. The projection image may be calculated for a projection of the minimum values or the maximum values in a particular voxel.

In another embodiment the breast micro-calcification image reconstruction module is implemented as an algorithm comprising the step of calculating a fibroglandular tissue segmentation of the DIXON magnetic resonance image data that identifies a location of fibroglandular tissue by inputting the DIXON magnetic resonance imaging data into a fibroglandular tissue identification module. The DIXON magnetic resonance imaging data may comprise a water and a fat image. As the breast is mostly adipose or fat tissue the water image will show predominantly the fibroglandular tissue.

The water image may therefore be readily segmented using an algorithm. The algorithm further comprises the step of calculating a fibroglandular tissue phase image of the fibroglandular tissue by inputting the fibroglandular tissue segmentation into a phase image calculation module. The phase image calculation module takes as input the location of tissue and then knowing the strength of the B0 field the likely perturbation to the field by the tissue can be calculated. It is known how to do this in several different ways. A so-called kernel calculation can be used as well as a trained neural network.

The algorithm further comprises the step of calculating a modified phase image by subtracting the fibroglandular tissue phase image from the gradient echo magnetic resonance imaging data. Performing this step essentially removes the effect of the fibroglandular tissue from the gradient echo magnetic resonance imaging data. Finally, the algorithm comprises the step of providing the breast micro-calcification magnetic resonance image by performing a phase unwrapping of the modified phase image. The phase in a normal phase image varies between - and + pi for example. When the phase transitions between one of these boundaries there will be an abrupt transition in the image. It is however, relatively straight forward to automatically perform a phase unwrapping and avoid this.

In another embodiment the fibroglandular tissue identification module is configured for providing the fibroglandular tissue segmentation by first thresholding the image then performing region growing, going through the following processes, not necessarily in the order mentioned - an erosion process, a dilation or smoothing process, to provide a smooth geometry of the segmentation.

In another embodiment the fibroglandular tissue identification module is configured for providing the fibroglandular tissue segmentation by inputting the DIXON magnetic resonance imaging data into a trained fibroglandular tissue identification neural network. For example, the DIXON magnetic resonance imaging data which may include the water and also in some cases the fat image into the trained fibroglandular tissue identification neural network which then outputs the result. This for example can be trained by producing specialized magnetic resonance imaging phantoms that have calcium particles placed within known locations of a magnetic resonance imaging breast phantom.

In another embodiment the phase image calculation module is implemented using a dipole kernel calculation module. This may be done using known methods of calculating a dipole kernel.

In another embodiment the phase image calculation module is implemented as a trained phase image-generating neural network. In this example the segmentation is input into the trained phase image generating neural network and, as an output, there is the modified phase image output. The training data for the trained phase image generating neural network may be produced in several different ways. In one case known tissue arrangements can be measured to calculate or provide the training data. In other examples a dipole kernel may be used to train the neural network.

In another embodiment the breast micro-calcification image reconstruction module is implemented as a trained breast micro-calcification image reconstruction neural network. In this example the entire steps of reconstructing the breast micro-calcification magnetic resonance image is performed by a single neural network. This neural network may be trained by providing sets of training data. Each set of the training data comprises the gradient echo magnetic resonance imaging data, the DIXON magnetic resonance imaging data and a control breast micro-calcification magnetic resonance image data. The breast micro-calcification magnetic resonance image could have for example been produced by performing measurements on a phantom. In other examples the breast micro-calcification magnetic resonance image may be produced algorithmically by analyzing the gradient echo magnetic resonance imaging data and the DIXON magnetic resonance imaging data.

In another embodiment execution of the machine-executable instructions further causes the computational system to receive k-space data acquired according to a DIXON magnetic resonance imaging data protocol and a gradient echo magnetic resonance imaging protocol with a single gradient echo or multiple gradient echoes. The k-space data could in some examples be combined, that is the DIXON magnetic resonance imaging data and the gradient echo magnetic resonance imaging data combined. The data could therefore be acquired in a single acquisition and this would ensure that they are spatially matched. In other examples the k-space data could have two portions, one which is the DIXON magnetic resonance imaging data and the other which is according to the gradient echo magnetic resonance imaging protocol.

Execution of the machine-executable instructions further causes the computational system to reconstruct the gradient echo magnetic resonance imaging data according to the gradient echo magnetic resonance imaging protocol. Execution of the machine-executable instructions further cause the processor to reconstruct the DIXON magnetic resonance imaging data according to the DIXON magnetic resonance imaging protocol.

In another embodiment the DIXON magnetic resonance imaging protocol is a compressed sensing protocol.

In another embodiment the gradient echo magnetic resonance imaging protocol is a compressed sensing protocol.

In another embodiment the DIXON magnetic resonance imaging protocol is a multi-point Dixon magnetic resonance imaging protocol. The use of a multi-point DIXON magnetic resonance imaging protocol may be beneficial because the resulting water and fat image or images may be more accurate which may lead to a more accurate identification of the location of breast micro-calcifications.

In another embodiment the medical system further comprises a magnetic resonance imaging system that is configured for acquiring k-space data from an imaging zone. The memory further contains pulse sequence commands configured for acquiring the measured k-space data according to the DIXON magnetic resonance imaging protocol and the gradient echo magnetic resonance imaging protocol. Execution of the machine-executable instructions further causes the computational system to acquire the k-space data by controlling the magnetic resonance imaging system with the pulse sequence commands. As was mentioned before, this may be a single acquisition of k-space data if the DIXON magnetic resonance imaging protocol and the gradient echo magnetic resonance imaging protocol are combined. In other examples it may be two separate acquisitions of k-space data if the protocols are performed separately.

In another embodiment the pulse sequence commands are configured for acquiring the k-space data for a region of interest. The pulse sequence commands are further configured for suppressing vascular structures by implementing at least one saturation band outside of the region of interest. The use of a saturation band may be useful because it can reduce or eliminate the MR signal coming from blood that is diffusing into the breast. As blood is mostly water, this will reduce the difficulty in correctly segmenting the fibroglandular tissue. The elimination of blood from the magnetic resonance images may also make it easier for a neural network to generate the breast micro-calcification magnetic resonance image.

In another embodiment the gradient echo magnetic resonance imaging has an echo time. The echo time is a long echo time.

In another embodiment the gradient echo magnetic resonance imaging data has an echo time. In this embodiment the echo time is between 100% and 90% of the T2* time for the fat tissue. In some examples this echo time may provide a definition of a long echo time. This embodiment may be beneficial because it may provide a means for identifying small breast micro-calcifications.

In another aspect the invention provides for a computer program comprising machine-executable instructions for execution by a computational system that is configured for controlling a medical system. Execution of the machine-executable instructions causes the computational system to receive a gradient echo magnetic resonance imaging data descriptive of a breast region of a subject. Execution of the machine-executable instructions further causes the computational system to receive DIXON magnetic resonance imaging data descriptive of the breast region. The DIXON magnetic resonance imaging data and the gradient echo magnetic resonance imaging data are spatially matched.

Execution of the machine-executable instructions further causes the computational system to generate the breast micro-calcification magnetic resonance image by inputting the gradient echo magnetic resonance imaging data and the DIXON magnetic resonance imaging data into a breast micro-calcification image reconstruction module. The breast micro-calcification image reconstruction module is configured to output a breast micro-calcification magnetic resonance image in response to inputting a gradient echo magnetic resonance imaging data and DIXON magnetic resonance imaging data. The breast micro-calcification phase magnetic resonance image is descriptive of a location of a breast micro-calcification. The gradient echo magnetic resonance imaging data is a phase image.

In another aspect the invention provides for a method of medical imaging. The method comprises receiving a gradient echo magnetic resonance imaging data descriptive of a breast region of the subject. The method further comprises receiving DIXON magnetic resonance imaging data descriptive of the breast region. The DIXON magnetic resonance imaging data and the gradient echo magnetic resonance imaging data are spatially matched. The method further comprises generating the breast micro-calcification magnetic resonance image by inputting the gradient echo magnetic resonance imaging data and the DIXON magnetic resonance imaging data into a breast micro-calcification image reconstruction module. The breast micro-calcification image reconstruction module is configured to output a breast micro-calcification magnetic resonance image in response to inputting a gradient echo magnetic resonance imaging data and DIXON magnetic resonance imaging data. The breast micro-calcification phase magnetic resonance image is descriptive of a location of a breast micro-calcification. The gradient echo magnetic resonance imaging data is a phase image.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

K-space data is defined herein as being the recorded measurements of radio frequency signals emitted by atomic spins using the antenna of a Magnetic resonance apparatus during a magnetic resonance imaging scan. Magnetic resonance data is an example of tomographic medical image data.

A Magnetic Resonance Imaging (MRI) image, MR image, or magnetic resonance imaging data is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of operating the medical system of Fig. 1; and
Fig. 3 illustrates a further example of a medical system.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

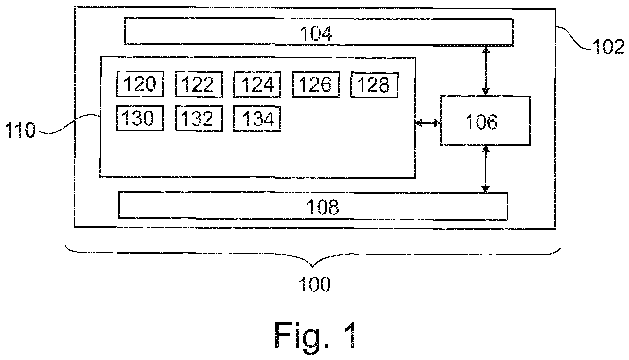

Fig. 1 illustrates an example of a medical system 100. The medical system 100 is shown as comprising a computational system 106 that in this example is part of a computer 102. The computational system 106 could however be embedded or incorporated into other devices or systems. The computer 102 is intended to represent one or more computers that may be networked or connected together. The medical system 100 could take different forms in different examples. In one example the medical system 100 could be a workstation or terminal that is controlling a magnetic resonance imaging system. In other examples the medical system 100 could be a computer or workstation used for the review of medical images. In another example the medical system 100 could be a remote computer that is providing cloud computing or other image processing services.

The computer 102 is further shown as containing a hardware interface 104 that is connected to the computational system 106. The hardware interface 104 may enable the computational system 106 to communicate with other computer systems and computational systems. In some examples the medical system 100 may comprise additional components such as a magnetic resonance imaging system and the hardware interface 104 may enable the computational system 106 to control the operation and function of the magnetic resonance imaging system. The computer 102 is further shown as containing a user interface 108 and a memory 110 that is also connected to the computational system 106. The user interface 108 may be an optional component that may enable an operator to use and control the medical system 100. The memory 110 is intended to represent any combination of memory that is accessible to the computational system 106. The memory 110 may in some examples be a non-transitory storage medium.

The memory 110 is shown as containing machine-executable instructions 120. The machine-executable instructions 120 enable the computational system 106 to control the operation and function of the medical system 100. The machine-executable instructions 120 may also enable the computational system 106 to perform various computational tasks, data processing tasks, as well as image processing tasks. For example, it may enable the computational system 106 to perform magnetic resonance imaging reconstructions.

The memory 110 is further shown as containing a breast micro-calcification image reconstruction module 122. The breast micro-calcification image reconstruction module 122 is configured for outputting a breast micro-calcification magnetic resonance image 128 in response to receiving gradient echo magnetic resonance imaging data 124 and DIXON magnetic resonance imaging data 126. In various examples the DIXON magnetic resonance imaging data 126 may be purely a water image. In other examples the DIXON magnetic resonance imaging data 126 comprises both a water and a fat image. The gradient echo magnetic resonance imaging data 124, the DIXON magnetic resonance imaging data 126 and the breast micro-calcification magnetic resonance image 128 are shown as being stored in the memory 110.

The breast micro-calcification image reconstruction module 122 may take different forms in different examples. It may be a purely algorithmic module, it may be a hybrid module that combines some algorithms with various AI components such as neural networks, or it may be implemented purely as a neural network.

The memory 110 is further shown as containing an optional high-pass spatial filter. The high-pass spatial filter is configured to enable objects which have small spatial features in comparison to the size of the image remain in the image where large features are removed. The high-pass spatial filter reduces the low spatial frequency content of the image. One way of implementing this would be to use a kernel of a particular size.

The memory 110 is further shown as containing a filtered breast micro-calcification image 132 that has been received in response to inputting the breast micro-calcification magnetic resonance image 128 through the high-pass spatial filter 130. This embodiment may be beneficial because it may possibly minimize or remove objects that are not micro-calcifications. Additionally, the use of a high-pass spatial filter helps simulate a mammogram. The memory 110 is further shown as containing a projection image 134. The projection image 134 is a projection of the filtered breast micro-calcification image 132 onto a two-dimensional plain. The filtered breast micro-calcification image 132 may for example be a three-dimensional magnetic resonance image or it may also be a collection of two-dimensional slices, which is also considered to be three-dimensional dataset. The projection image 134 may be useful for radiologists who have been accustomed and trained to interpret mammograms. The combination of the high-pass spatial filter and the production of the projection image 134 produces a magnetic resonance image that is suitable for substituting for a mammogram.

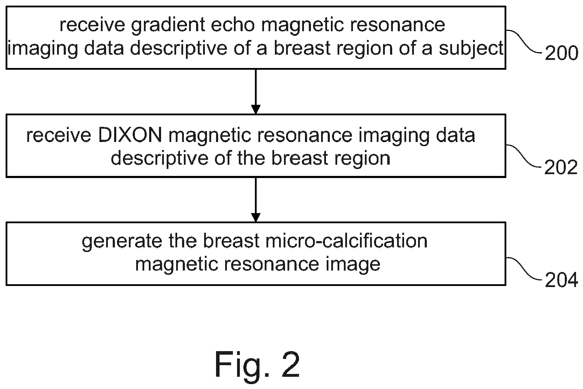

Fig. 2 shows a flowchart which illustrates a method of operating the medical system 100 of Fig. 1. First in step 200 the gradient echo magnetic resonance imaging data 124 is received. Next in step 202 the DIXON magnetic resonance imaging data 126 is received. This DIXON magnetic resonance imaging data 126 is also descriptive of the breast region. The breast region may for example be a region of interest in the magnetic resonance images. The DIXON magnetic resonance imaging data 126 and the gradient echo magnetic resonance imaging data 124 are spatially matched. Finally, in step 204, the breast micro-calcification magnetic resonance image 128 is generated by inputting the gradient echo magnetic resonance imaging data 124 and the DIXON magnetic resonance imaging data 126 into the breast micro-calcification image reconstruction module 122.

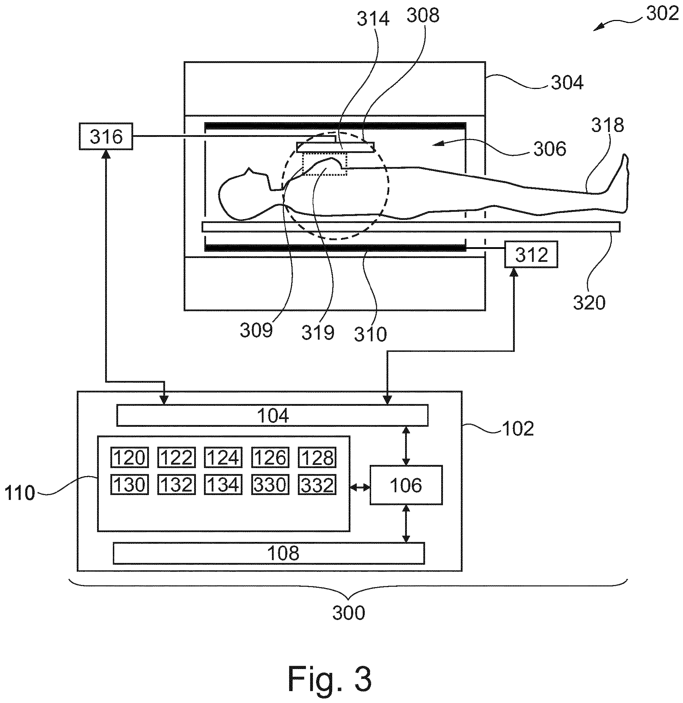

Fig. 3 illustrates a further example of a medical system 300. The medical system 300 depicted in Fig. 3 is similar to the medical system 100 in Fig. 1 except the medical system additionally comprises a magnetic resonance imaging system 302.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically acquried for the region of interest. A subject 318 is shown as being supported by a subject support 320 such that at least a portion of the subject 318 is within the imaging zone 308 and the region of interest 309. The region of interest 309 is shown as having breast region or breast tissue 319 within it.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the gradient controller 312 are shown as being connected to the hardware interface 106 of a computer system 102.

The memory 110 is further shown as containing pulse sequence commands 330. The pulse sequence commands contain data or instructions which may be used to control the magnetic resonance imaging system 302 to acquire k-space data 332 according to a DIXON magnetic resonance imaging protocol and a gradient echo magnetic resonance imaging protocol. The memory 110 is further shown as containing k-space data 332 that has been acquired by controlling the magnetic resonance imaging system 302 with the pulse sequence commands 330.

Breast micro-calcification are one of the early precursors of breast cancer. While Mammography, is the currently preferred diagnostic mode for microcalcification detection, it uses ionizing X-ray radiation. Reliable detection of breast microcalcification using MR, would provide a safer alternative for breast cancer screening. Calcification found in-vivo has a distinct magnetic susceptibility compared to surrounding tissue. MRI signal phase can be sensitive to such susceptibility changes. Presence of fat, that also affects MRI signal's phase, poses a confounding factor. In the method proposed, the contribution of fat signal is removed using prior information from mDIXON or other types of DIXONS scans to create a relatively clean phase image with sensitivity primarily to the presence of breast calcification. This method would be a strong enabler towards MRI based breast cancer screening, as it addresses one of the key pit-falls of MR - namely, inability to image micro-calcification.

Breast cancer is the 2nd most common form of cancer worldwide and the leading cause of cancer in women. Mammography is the standard first line of diagnostic imaging for breast cancer screening. The European Society of Medical Oncology, the American Cancer Society as well as the Indian Council for Medical Research recommend annual breast screening using mammography for women over 45 years of age and/or who have higher familial risk of breast cancer. In approximately 31% of the patients undergoing a screening mammogram, breast micro-calcifications are observed, which are associated with ductal carcinoma in-situ (DCIS), a potential precursor of invasive breast cancer. Since not all DCIS patients migrate to invasive cancer stage, detection of micro-calcifications in an initial screening mammogram typically prompts follow up mammography or magnetic resonance imaging (MRI) based assessment. Mammography procedure utilizes ionizing radiation for imaging purposes, which itself is an important carcinogenic risk factor. Annual screening mammography examinations further increase exposure to ionizing radiation. MRI, on the other hand, provides a non-ionizing radiation based imaging alternative for breast cancer assessment. MRI, on the other hand, provides a non-ionizing radiation based imaging alternative for breast cancer assessment. Nevertheless, one of the important drawbacks in the use of MRI for generic breast cancer screening is its inability to definitively detect micro-calcifications, which are one of the early imaging markers breast cancer.

Current proposed MRI methods utilize either phase based pattern matching or quantitative susceptibility mapping based methods for detecting breast calcification. Most of these methods have not been used in-vivo either due to long scan times or have not been used for detecting near micro or micro-calcification. They have been used for visualizing large breast calcification. Quantitative susceptibility mapping (QSM) based method has been used for detecting large breast calcification previously. QSM is a neat tool that accounts for the phase effects of fibroglandular tissue, and of fat and can help in visualizing calcification nodules. However, QSM has not been proven to be useful in detecting micro-calcification. In this work, we propose an optimized MRI protocol and a post-processing algorithm that uses MRI directly, for the detection of breast-calcifications.

Breast calcifications are nodular and typically partial volume relative to the imaging voxel size due to relative size differences (microcalcifications are on the order of 0.1-0.4mm in diameter and the typical imaging voxel sizes are on the order of 2 mm in slice thickness). The partial volume phase will be detectable in gradient echo MRI phase images and can be identified as nodules using projection images, since vessels and fibroglandular tissue are contiguous structures. Fibroglandular tissue will also have significant phase effect due to its susceptibility difference between itself and the surrounding breast fat. This phase effect can be mitigated if the geometry of the fibroglandular tissue is known apriori. This information can be obtained from an mDIXON or other DIXON technique, whose implementation is unique to Philips. The phase from fibroglandular tissue can then be estimated using a generic dipole kernel. Using this simulated phase information, a clean breast phase image with sensitivity only to calcification nodules can then be obtained.

Visualization of breast calcification in phase images has been done previously. Likewise, using tissue geometry to predict phase information has been done before in the case of brain, where tissue geometry was extracted from a different acquisition. The novel aspect of this invention/innovation is - the unique combination of a gradient echo acquisition along with mDIXON or other DIXON acquisition for the application of breast calcification detection.

A method of detecting breast calcifications may comprise one or more of the following steps:
1) Acquisition of long echo time (TE) gradient echo data and mDIXON or other DIXON data with matching resolutions and bandwidths - either in one single sequence or as separate acquisitions. The long echo time (TE) gradient echo data may be the gradient echo magnetic resonance imaging data 124, and may also be simply referred to as echo time (TE) gradient echo data without the "long TE" label. The mDIXON or other DIXON data may be the DIXON magnetic resonance imaging data 126.
2) Compressed SENSE may be used for faster acquisition of high resolution data in some examples, but is not necessary. In some examples, the voxel size is on the order of 0.1 x 0.1 mm2 to 0.4 x 0.4 mm2 following voxel aspect rations on the order of 1:1:3 to 1:1:5.
3) The long TE gradient echo data is acquired with a TE that is in-phase with fat. There are advantages to being in-phase with fat as mentioned above, but the phase can be freely chosen.
4) Obtaining geometry of fibroglandular tissue (a fibroglandular tissue segmentation) from mDIXON images using image processing methods including, but not limited to, thresholding, region growing, erosion and dilation for smooth geometry extraction. This is a fibroglandular tissue segmentation of the DIXON magnetic resonance imaging data 126.
5) Elimination of vascular structures within the mDIXON images may be performed if needed, either using a saturation band during data acquisition, or through vessel tracking means in data acquired without saturation.
6) Simulate the phase effects of fibroglandular tissue using one of the dipole kernels or using a machine learning approach.
7) Removing phase effects of fibroglandular tissue from the long TE phase images through phase subtraction.
8) Unwrapping the phase.
9) Filtering the phase images (with a high-pass spatial filter) for removing low frequency background field information.
10) Creating projection images 134 (maximum or minimum intensity projection depending on the handedness of the phase images) for the visualization of nodular breast calcifications.
11) An alternative implementation may use a machine learning framework (for example a neural network) with phase and geometry information as inputs and the output being projection images showing only calcification nodules.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: medical system
- 102: computer
- 104: hardware interface
- 106: computational system
- 108: user interface
- 110: memory
- 120: machine executable instructions
- 122: breast micro-calcification image reconstruction module
- 124: gradient echo magnetic resonance imaging data
- 126: DIXON magnetic reonance imaging data
- 128: breast micro-calcification magnetic resonance image
- 130: high-pass spatial filter
- 132: filtered breast micro-calcification image
- 134: projeciton image
- 200: receive gradient echo magnetic resonance imaging data descriptive of a breast region of a subject
- 202: receive DIXON magnetic resonance imaging data descriptive of the breast region
- 204: generate the breast micro-calcification magnetic resonance image by inputting the gradient echo magnetic resonance imaging data and the DIXON magnetic resonance imaging data into the breast micro-calcification image reconstruction module
- 300: medical system
- 302: magnetic resonance imaging system
- 304: magnet
- 306: bore of magnet
- 308: imaging zone
- 309: region of interest
- 310: magnetic field gradient coils
- 312: magnetic field gradient coil power supply
- 314: radio-frequency coil
- 316: transceiver
- 318: subject
- 319: breast region
- 320: subject support
- 330: pulse sequence commands
- 332: k-space data

## Claims

1. A medical system (100, 300) comprising:
- a memory (110) storing machine executable instructions (120) and a breast micro-calcification image reconstruction module (122), wherein the breast micro-calcification image reconstruction module is configured to output a breast micro-calcification magnetic resonance image (128) in response to inputting gradient echo magnetic resonance imaging data (124) and DIXON magnetic resonance imaging data (126), wherein the breast micro-calcification magnetic resonance image is descriptive of a location of breast micro-calcifications, wherein the gradient echo magnetic resonance imaging data is a phase image; and
- a computational system configured for controlling the medical system, wherein execution of the machine executable instructions causes the computational system to:
- receive (200) the gradient echo magnetic resonance imaging data descriptive of a breast region (318) of a subject (318);
- receive (202) the DIXON magnetic resonance imaging data descriptive of the breast region, wherein the DIXON magnetic resonance imaging data and the gradient echo magnetic resonance imaging data are spatially matched; and
- generate (204) the breast micro-calcification magnetic resonance image by inputting the gradient echo magnetic resonance imaging data and the DIXON magnetic resonance imaging data into the breast micro-calcification image reconstruction module.

2. The medical system of claim 1, wherein execution of the machine executable instructions further causes the computational system to calculate a filtered breast micro-calcification image (132) by applying a high-pass spatial filter (130) to the breast micro-calcification magnetic resonance image.

3. The medical system of claim 2, wherein execution of the machine executable instructions further causes the computational system to generate a breast micro-calcification projection image (134) by calculating a projection of the filtered breast micro-calcification image.

4. The medical system of claim 1, 2, or 3, wherein the breast micro-calcification image reconstruction module is implemented an algorithm comprising the steps of:
- calculate a fibroglandular tissue segmentation of the DIXON magnetic resonance imaging data that identifies a location of fibroglandular tissue by imputing the DIXON magnetic resonance imaging data into a fibroglandular tissue identification module;
- calculate a fibroglandular tissue phase image of the fibroglandular tissue by inputting the fibroglandular tissue segmentation into a phase image calculation module;
- calculate a modified phase image by subtracting the fibroglandular tissue phase image from the gradient echo magnetic resonance imaging data; and
- provide the breast micro-calcification magnetic resonance image performing a phase unwrapping of the modified phase image.

5. The medical system of claim 4, wherein the fibroglandular tissue identification module is configured for providing the fibroglandular tissue segmentation by any one of the following:
- thresholding, region growing, erosion, and dilation for smooth geometry extraction; and
- inputting the DIXON magnetic resonance imaging data into a trained fibroglandular tissue identification neural network; and
- combinations thereof.

6. The medical system of claim 4 or 5, wherein the phase image calculation module is implemented as any one of the following:
- using a dipole kernel calculation module; and
- a trained phase image generating neural network.

7. The medical system of claim 1, 2, or 3, wherein the breast micro-calcification image reconstruction module is implemented as a trained breast micro-calcification image reconstruction neural network.

8. The medical system of any one of the preceding claims, wherein execution of the machine executable instructions further causes the computational system to:
- receive k-space data (332) acquired according to a DIXON magnetic resonance imaging protocol and a gradient echo magnetic resonance imaging protocol with a single gradient echo or multiple gradient echoes;
- reconstruct the gradient echo magnetic resonance imaging data according to the gradient echo magnetic resonance imaging protocol; and
- reconstruct the DIXON magnetic resonance image data according to the DIXON magnetic resonance imaging protocol.

9. The medical system of claim 8, wherein the DIXON magnetic resonance imaging protocol is a compressed sensing protocol and/or the gradient echo magnetic resonance imaging protocol is a compressed sensing protocol.

10. The medical system of claim 8 or 9, wherein the DIXON magnetic resonance imaging protocol is a multi-point DIXON magnetic resonance imaging protocol.

11. The medical system of claim 8, 9, or 10, wherein the medical system further comprises a magnetic resonance imaging system (302) configure for acquiring k-space data from an imaging zone (109), wherein the memory further contains pulse sequence commands (330) configured for acquiring the measured k-space acquired according to the DIXON magnetic resonance imaging protocol and the gradient echo magnetic resonance imaging protocol, wherein execution of the machine executable instructions further causes the computational system to acquire the k-space data by controlling the magnetic resonance imaging system with the pulse sequence commands.

12. The medical system of claim 11, wherein the pulse sequence commands are configured for acquiring the k-space for a region of interest (109), wherein the pulse sequence commands are further configured for suppressing vascular structures by implementing at least one saturation band outside of the region of interest.

13. The medical system of any one of claims 8 through 12, wherein the gradient echo magnetic resonance imaging data has an echo time, wherein the echo time is any one of the following: a long echo time and the echo time is between 100% and 90% of the T2* time for fat tissue.

14. A computer program comprising machine executable instructions (120) for execution by a computational system (106) configured for controlling a medical system (100, 300),
wherein execution of the machine executable instructions causes the computational system to:
- receive (200) gradient echo magnetic resonance imaging data (124) descriptive of a breast region (319) of a subject (318);
- receive (202) DIXON magnetic resonance imaging data (126) descriptive of the breast region, wherein the DIXON magnetic resonance imaging data and the gradient echo magnetic resonance imaging data are spatially matched; and
- generate (204) a breast micro-calcification magnetic resonance image (122) by inputting the gradient echo magnetic resonance imaging data and the DIXON magnetic resonance imaging data into a breast micro-calcification image reconstruction module (128), wherein the breast micro-calcification image reconstruction module is configured to output a breast micro-calcification magnetic resonance image in response to inputting the gradient echo magnetic resonance imaging data and the DIXON magnetic resonance imaging data, wherein the breast micro-calcification magnetic resonance image is descriptive of a location of breast micro-calcifications, wherein the gradient echo magnetic resonance imaging data is a phase image.

15. A method of medical imaging, wherein the method comprises:
- receiving (202) gradient echo magnetic resonance imaging data (123) descriptive of a breast region (319) of a subject (318);
- receiving (202) DIXON magnetic resonance imaging data descriptive of the breast region, wherein the DIXON magnetic resonance imaging data and the gradient echo magnetic resonance imaging data are spatially matched; and
- generating (204) a breast micro-calcification magnetic resonance image (128) by inputting the gradient echo magnetic resonance imaging data and the DIXON magnetic resonance imaging data into a breast micro-calcification image reconstruction module (122), wherein the breast micro-calcification image reconstruction module is configured to output a breast micro-calcification magnetic resonance image in response to inputting the gradient echo magnetic resonance imaging data and the DIXON magnetic resonance imaging data, wherein the breast micro-calcification magnetic resonance image is descriptive of a location of breast micro-calcifications, wherein the gradient echo magnetic resonance imaging data is a phase image.
